# EUROPEAN PATENT APPLICATION

(11) **EP 1 988 480 A1**
(43) Date of publication of application: **05.11.2008**
(21) Application number: 07103821.0
(22) Date of filing: 09.03.2007
(51) Int. Cl.: G06F 19/00

(54) **Computer program product, method and device for guiding the breathing**

(71) Applicant: Koninklijke Philips Electronics N.V., 5621 BA Eindhoven (NL)
(72) Inventor: The designation of the inventor has not yet been filed
(74) Representative: Schoenmaker, Maarten

(57) **Abstract**

Breathing guide for guiding the breathing of a user, provided with a heart beat sensor configured to convert a human or animal heart beat into heart beat signals, a storage arrangement configured to store an algorithm, a processing circuit configured to process heart beat signals and breathing guidance signals, a user interface configured to indicate a breathing guidance signal, and an algorithm configured to convert the heart beat signals into breathing guidance signals and to synchronize the breathing guidance signals with the processed heart rate signals for the purpose of guiding the user towards an improved heart coherence.

## Description

### FIELD OF THE INVENTION

The invention relates to a computer program product for guiding the breathing of a user.

The invention also relates to a method for guiding the breathing of a user.

Furthermore the invention relates to a breathing guide for guiding the breathing of a user.

Heart rate (or heart beat rate) variability is the irregularity in the heart rate during a specific period, i.e. the variability of the time intervals between individual beats. Having a high heart rate variability (also called heart variability) is considered to indicate a healthy flexibility of one's parasympathetic system.

### BACKGROUND OF THE INVENTION

In the market products are known that relate to the heart rate variability, such as the 'Freeze-Framer®' product of the company HeartMath LLC. In particular, this product claims to improve the health by improving the heart coherence. Heart coherence is related to the heart rate variability. Generally, a high heart rate variability indicates a high heart coherence score. In the art, heart coherence is said to improve by combining intentional heart focus (e.g. with the aid of slow breathing) combined with positive feelings.

In order to measure heart coherence, in the Freeze-Framer® product the heart rate is measured by measuring a pulse wave with a finger plethysmograph. The heart beat signals that are derived are processed such that changes in the heart rate can be shown in a graph. From this graph the heart rate variability can be derived. Also the level of heart coherence in time is indicated on screen with the Freeze-Framer® product.

It is also known to influence the heart beat rhythm by controlling the breathing rhythm. For example, to improve the heart coherence the Freeze-Framer® product asks the user to breathe 'through the heart', and for example tries to stimulates this in some way with music. Other products claim to lower blood pressure by helping the user to breathe slowly, deep and/or regularly. This principle is for example applied in Yoga practice. Oftentimes, the breathing process towards an improved heart coherence is guided by an expert.

However, these known ways and products are not sufficiently suitable for guiding the user, who may not be an expert, towards an initial heart coherence and/or heart rate variability. Also these products are not a sufficient aid in maintaining and/or improving the heart coherence and/or heart rate variability.

### SUMMARY OF THE INVENTION

Therefore, it is a goal of the invention to guide a user towards a heart coherence and/or heart rate variability, preferably in a relatively easy and/or intuitive way.

Another goal is to maintain and/or improve the heart coherence and/or heart rate variability, preferably in a relatively easy and/or intuitive way.

In a first aspect, at least one of said goals and/or other goals can be achieved individually or in combination, having no preferred hierarchy, by a computer program product for guiding the breathing of a user, which when executed on a computer controls the computer to process received heart beat signals and to provide for breathing guidance signals via a user interface, wherein the computer program product comprises an algorithm that is configured to synchronize the breathing guidance signals with the received heart beat signals for the purpose of guiding the user towards an improved heart coherence and/or heart rate variability.

Such a computer program product will allow a breathing rhythm of the user that is relatively directly related to the heart beat, which will improve the heart coherence and/or heart rate variability. The breathing guidance signals will signal the user to breathe in and/or out in sync with heart beats, such that a heart coherence and/or heart rate variability can be reached, and preferably maintained and/or improved, in a relatively easy and preferably intuitive way. No specialist, training and/or extra knowledge are needed for the user, to achieve a heart coherence and/or heart rate variability. By indicating only the breathing guidance signals the user may focus entirely on his or her breathing, without being distracted by the heart beat (signals). In particular embodiments, the computer program, when executed on a computer, functions as a self-adjusting automated breathing guide, such that with relatively little effort and/or intuitively a user that is not an expert may improve his or her heart coherence and/or heart rate variability.

In a second aspect, at least one of said goals and/or other goals can be achieved individually or in combination, having no preferred hierarchy, by a method for guiding the breathing of a user, wherein heart beat signals are measured in an automated way, wherein breathing guidance signals that are synchronized with heart rate signals are given in an automated way for the purpose of guiding the user towards an improved heart coherence and/or heart rate variability.

In a third aspect, at least one of said goals and/or other goals can be achieved individually or in combination, having no preferred hierarchy, by a breathing guide for guiding the breathing of a user, provided with a heart beat sensor configured to convert a human or animal heart beat into heart beat signals, a storage arrangement configured to store an algorithm, a processing circuit configured to process heart beat signals and breathing guidance signals, a user interface configured to indicate a breathing guidance signal, wherein the algorithm is configured to convert the heart beat signals into breathing guidance signals and to synchronize the breathing guidance signals with the processed heart rate signals for the purpose of guiding the user towards an improved heart coherence and/or heart rate variability.

### BRIEF DESCRIPTION OF THE DRAWINGS

In clarification of the invention, embodiments thereof will be further elucidated with reference to the drawing. In the drawing:
Fig. 1 schematically shows a breathing guide;
Figs. 2A and 2B show heart coherence scores and heart rates, respectively, against time;
Figs. 3A-C show methods of steps of increasing a dividing constant during certain time intervals;
Fig. 4 shows a self-adjusting feature of the breathing guide;
Fig. 5 shows a breathing guide.

### DETAILED DESCRIPTION OF PREFERRED EMBODIMENTS

In this description, identical or corresponding parts have identical or corresponding reference numerals. The exemplary embodiments shown should not be construed to be limitative in any manner and serve merely as illustration.

In this description, heart beat signal frequency is related to the heart rate, wherein the heart beat signal frequency refers to the frequency of the measured signals while the heart rate refers to the actual heart rate of a human or animal. Ideally, the noise of the heart rate signals is reduced in such a way that the heart rate of a person and his or her measured heart beat signal frequency are the same. Because of the heart rate variability the intervals between individual heart beats change continuously, such that the heart rate, i.e. the heart beat signal frequency, also changes continuously. This (varying) rate or frequency can be calculated in several ways as will become clear from this description. For example, in an embodiment, the average heart signal frequency over the last five heart beats is calculated and shown in a graph.

The 'breathing guidance signal frequency' is related to the frequency of providing a breathing signal. For example, one signal can be given to a user 2 for breathing in and again one signal for breathing out. The breathing rhythm however is the frequency of performing one breath, wherein one breath refers to one time breathing in and one time breathing out. For example, if a user follows the breathing guidance signals that are given one time for breathing in and one time for breathing out, the breathing guidance signal frequency is double the breathing rhythm of the user. Breathing guidance signals could also be given just for breathing in or just for breathing out, wherein the breathing rhythm and the breathing guidance signal frequency could be the same.

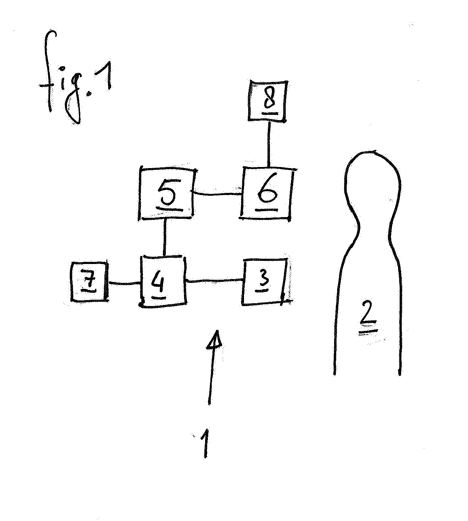

In Figure 1, a breathing guide 1 for guiding the breathing of a user 2 is shown. The breathing guide 1 is provided with a heart beat sensor 3 configured to derive heart beat signals from a human or animal heart beat. These signals are processed by a processing circuit 4 with the aid of a programmed algorithm stored in a storage arrangement 5. A user interface 6 is configured to indicate breathing guidance signals to the user 2. These elements 3, 4, 5, 6 can be connected to each other in a wired, wireless or integrated way and can be fed by any suitable power supply 7. Optionally, a user setting interface 8 is provided, for example as a part of the user interface 6, such that settings for each user 2 can be set and/or changed, for example by a specialist or the user 2 himself. This user setting interface 8 may for example comprise a personal computer or the like.

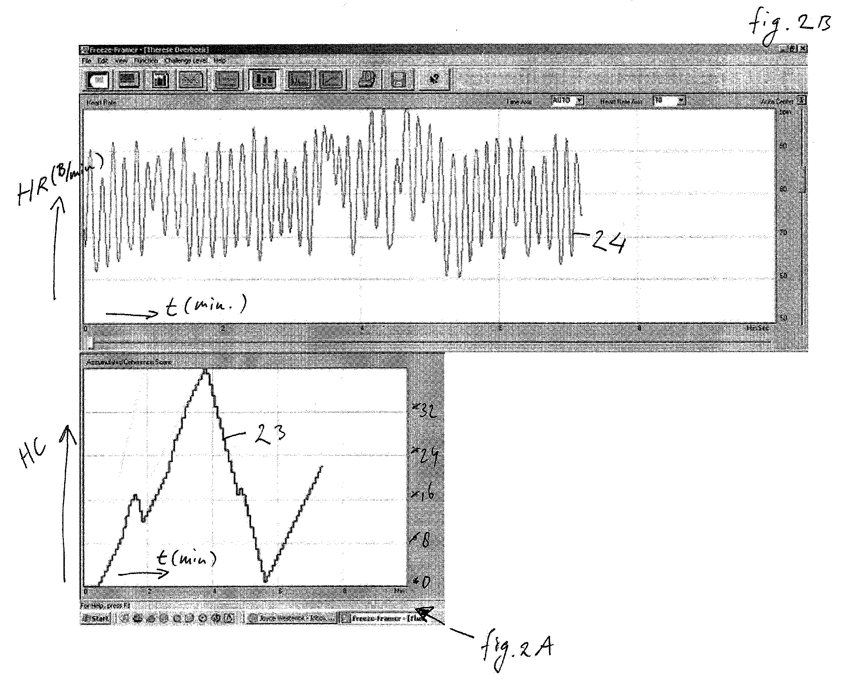

It is found that if one breathes slowly while the breathing is synchronized with the heart beat, a better heart coherence is reached than if one breathes slowly while the breathing is not synchronized with the heart beat. The positive effect of breathing in synchronization with the heart beat is illustrated in Figure 2A and/or 2B, showing the results of the test that were rendered by the Freeze framer® product of HeartMath. In Figure 2A, the horizontal axis shows the time in minutes t(min) and the vertical axis shows the heart coherence score, indicated in the figure by HC. Here, the heart coherence score is a variable calculated by certain algorithms that are defined in the Freeze framer® software. Individually, 2, 4, 6 and 8 minutes are indicated at the horizontal axis wherein the graph stops at approximately 7 minutes. In Figure 2B, the horizontal axis also shows the time in minutes, indicated by t(min), and the vertical axis shows the heart rate in beats per minute, indicated by HR(b/min).

In this test a person was guided to breathe in over 6 heart beats and breathe out over 6 heart beats. This was done during a first time interval, in this illustrative example between 2 and 4 minutes, and during a third time interval, in this illustrative example between 5.5 and 7 minutes (see Figure 2A). During a second time interval, in this illustrative example between 4 and 5.5 minutes, a steady, relatively slow breathing rhythm was indicated, that was unrelated to the heart beat. The heart coherence scores, as Figure 2A shows by the inclining heart coherence graph 23, went up when the breathing was brought in synchronization with the heart beat. During the first interval, between 2 and 4 minutes, approximately, and during the third time interval, between 5.5 and 7 minutes, approximately, the heart coherence graph 23 clearly inclines, indicating a high coherence. For the same first and third interval, the variability of the heart rate, indicated with graph 24, was relatively high, as can be seen from Figure 2B, wherein the variability graph 24 shows relatively large distances between the peaks and the valleys. These distances indicate a relatively large difference between the highest and lowest hart rate, which, in turn, indicates a high variability. Hence heart coherence and heart rate variability are related. More importantly, it was shown that bringing the breathing rhythm in sync with the heart beat, in this case breathing in and out, respectively, at every sixth heart beat, has a positive effect on heart coherence and heart rate variability.

As can be seen from Figure 2A, during the second time interval, which was between 4 and 5,5 minutes, approximately, the heart coherence graph 23 was declining, indicating a relatively lower coherence score. This corresponds with Figure 2B, wherein during the second interval, the distances between the peaks and valleys of the variability graph 24 are on average decreasing, indicating a relatively low heart rat variability.

It is clear that when breathing in synchronization with the heart beat, the heart beat can be influenced and high heart rate variabilities can be reached. However, breathing in synchronization with the heart beat would require silence and concentration from the user, and even then some people cannot even feel their own heart beat. Therefore, the algorithm that is stored in the storage arrangement 2 is configured to convert the heart beat signals that are received from the heart beat sensor 3 into breathing guidance signals such that the breathing guidance signals are synchronized with the processed heart rate signals. The breathing guidance signals are then perceived by the user 2 such that he or she will breathe in or out on the breathing guidance signal. In this way, the user 2 is guided to breathe synchronically with the heart beat such that an improved heart coherence and/or variability can be reached, maintained and/or improved without needing to be very occupied with his or her heart beat. The breathing guidance signals are preferably communicated in such a way that the user may synchronize its breathing in a relatively easy and intuitive way.

For measuring the heart beat and converting it into heart beat signals, several heart sensors 3 can be applied. As an illustrative example, the heart rate, or pulse, may be measured with a plethysmograph for example using photodiode or impedance measuring techniques. Obviously other sensors 3 are just as well suitable within the scope of the invention.

The user interface 6 is configured to indicate the user 2 to breathe in and to breathe out. Preferably this is indicated in a non-intrusive way that is easy to interpret, more particularly that can be interpreted in an intuitive way by the user 2. For example, the user interface 6 could comprise a vibrating element that is attached to the user's body, or one or a series of LED's could be used to indicate breathing guidance signals. Also, any type of LCD or TFT screen or the like could be suitable. In principle, any signal that can be interpreted by a user 2, such as a sound, visual or other sensory signal, may be used to communicate a breathing signal to the user 2, such that multiple user interfaces 6 are suitable.

A breathing guide 1 has a storage arrangement 5 for storing a computer program product. The computer program product may be configured for guiding the breathing of the user. An algorithm that is configured to synchronize the breathing guidance signals with the heart beat signals, is programmed in the computer program product, for the purpose of guiding the user towards a high(er) heart coherence and/or heart rate variability.

The computer program product may in principle be executed on any computer such that in connection with a heart sensor 3, breathing guidance signals can be presented on screen. Also a specially configured breathing guide 1 device can be arranged to run the computer program. Obviously, in multiple ways, a convenient method for guiding the breathing of a user can be realized. For convenience as well as precision reasons, the heart beat signals are measured in an automated and preferably non-obtrusive way, and the breathing guidance signals that are synchronized with heart rate signals are given in an automated and preferably intuitively perceivable way.

Said algorithm may be configured to calculate the timing of the breathing guidance signal by dividing the heart beat signal frequency by a number, defined by dividing constant N. Preferably, N is an integer number. For example, the guidance signal timing could be calculated by dividing the heart beat frequency by 4 (N = 4). Then, if one has a heart beat signal frequency of 72 beats per minute, a breathing guidance signal frequency of 18 is calculated, implying that the user should alternately breathe in and breathe out each 9 times per minute. Thus, N is the ratio between the heart beat signal frequency and the breathing guidance signal frequency. Thus in this example, the user has a breathing rhythm of 9 breaths per minute. For example, heart rates may vary from 50 to 180 beats per minute, while breathing rhythms may for example vary from 2 to 15 breaths per minute (guidance signal frequencies may for example be twice as much). For example, when a user 2 starts using a breathing guide 1, he could start with a relatively low N, e.g. N = 2 or 3, to breathe in sync with every 2^{nd} or 3^{rd} heart beat, which will be relatively easy. Gradually the device 1 may increase N, so that gradually the breathing guidance signal frequency, and hence the breathing rhythm, is decreased and the variability may be increased. Of course, this increasing of N will not go on infinitely and depending on the calculated heart variability results, at a certain moment N will stabilize, and/or decrease. In most practical cases, during use, N will be less than 45 and will most probably lie between 12 and 2.

In an embodiment of a method that is convenient for the user 2, the breathing guidance signal frequency is approximately 15 breaths per minute, for example. N can be chosen such that a breathing guidance signal frequency of approximately 15 is achieved. Other convenient breathing guidance signal frequencies, for which N can be calculated, may for example be between 10 and 18 breaths per minute. In other words, N is calculated to provide for a convenient breathing rhythm for the user 2, for example such that heart coherence and/or heart rate variability can be improved and/or upheld during relatively long periods.

In an embodiment the breathing guidance is synchronized with the individual heart beats. For this, the algorithm is configured to indicate the breathing guidance signal on every Nth heart beat signal. For example, when N = 4, a breathing guidance signal is given on every fourth heart beat, wherein a signal indicates to breathe in and the next signal to breathe out. This will provide for a direct way of signaling the user and an early coherence can be achieved. This method has proven to be relatively effective and precise, because the breathing may be guided exactly in sync with one's heart beat, while it may be suitable for any (non-expert) user.

In another embodiment, the heart beat signal frequency is the average heart beat frequency calculated over a predetermined number of previous heart beat signals, for example the previous five heart beat signals. This may be advantageous because the heart beat signals are usually slightly distorted by noise. Also, the time intervals between individual beats will vary slightly. Calculating an average over the last previous heart beats may then be advantageous. For example, the average heart beat frequency over the last 5 heart beats can be calculated and divided by N in order to calculate the breathing guidance frequency. Instead of 5, the average heart beat frequency could for example be calculated over the last 20 or over the last three heart beats, or somewhere in between. In this embodiment a relatively high regularity of the breathing guidance signal frequency may be reached whereas at the same time the breathing guidance signals will be approximately in synchronization with the heart beat. Also other statistical methods could be applied over the last series of heart beats to even out distortion and synchronize heart beat and breathing.

Furthermore, other signal processing techniques may be used. For example, a heart beat signal pattern can be plotted in time, from which a breathing rhythm of a user 2 can be calculated. For example when there already is a high heart coherence and/or high heart rate variability the speeding up and slowing down of the heart rate is already related to the breathing rhythm of the user 2. Consequently, the breathing guidance signal frequency of the computer program can be synchronized with the breathing rhythm of the user 2 such that coherence and/or variability is upheld or improved. Therefore, N is calculated such that the breathing guidance signals are synchronized with the calculated breathing rhythm. This allows for relatively steady, easy and/or personally adapted breathing in sync with the user's heart beats. For example, a heart beat rate pattern 24 in time can be provided, see for example the graph 24 of Figure 2B, from which high and low points on the graph can be derived that correspond to the beginning of breathing in and the beginning of breathing out, respectively, of the user 2. N can then be calculated such that the breathing guidance signals are at least approximately synchronized with the breathing rhythm of the user 2.

To guide the user 2 towards a slow breathing rhythm, an algorithm can be applied such that the breathing guidance signal frequency is gradually decreased in time. For this, N is gradually increased in time. This may allow the user 2 to reach a slow as well as synchronized breathing rhythm. In this way the heart coherence and/or heart rate variability may be improved gradually and intuitively.

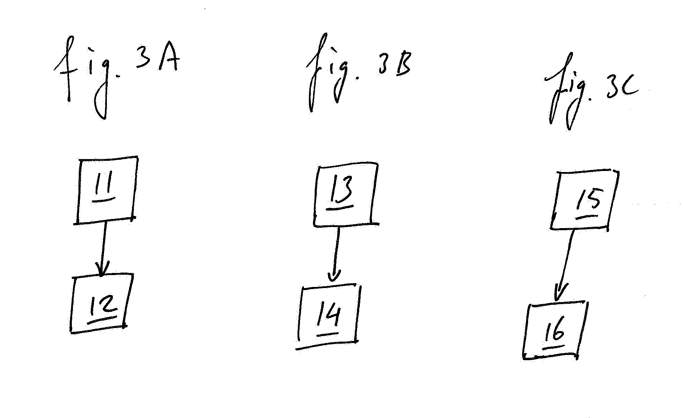

In an embodiment, to actively guide the user 2, the algorithm is configured such that the value of N is increased (e.g. to N+1) and stored in steps and the time intervals between those steps are stored in the storage arrangement 5 of the breathing guide 1. When the user 2 uses the breathing guide 1 at a later time of usage, applying the same dividing constant N as before, N is increased again, but now by a larger value than the previous time (e.g. N+2), such that the process is sped up. An example of this self adjusting feature is illustrated in Figure 3A and 3B. Figure 3A illustrates a first time of usage and Figure 3B a second time of usage of a breathing guide 1. In Figure 3A, a first step 11 indicates a time interval t1 of 60 seconds wherein N=N. A next step 12 indicates a second time interval t2, also of 60 seconds, wherein N is increased with a value of 1, such that N=N+1. For the second time of usage, the first step 13 also indicates a time interval t1 of 60 seconds for N=N and at the next step 14, t2 is also 60 second, but N is enlarged with a value of 2, such that N = N+2. This means that N has increased with a larger value for a corresponding time interval, with respect to the previous step (Figure 3A). Of course the values of increasing N and time intervals may vary.

In a like self-adjusting feature, after a first time of usage (e.g. Figure 3A), at a later time of usage N is increased again with the same value, e.g. from N to N+1, but within a shorter time interval than a first usage, e.g. the second time interval t1 could be 50 seconds instead of 60 seconds. This is illustrated in Figure 3C, which refers to a second usage after a first usage that is indicated in Figure 3A. In the illustrative example of Figure 3C, at step 15, t1 is 50 seconds and N = N, and at step 16, t2=45 seconds and N=N+1. According to the principles of Figure 3A- 3C the breathing of a user 2 may be slowed down more rapidly and more optimal heart coherence and/or heart rate variability can be achieved relatively more rapidly in an automated way.

In another embodiment, for example, said increasing value may be increased and said time interval may be shortened at the same time. For example t2=45 seconds and N=N+2, slowing down breathing even more rapidly.

To provide for a safe, effective and/or convenient system, it should be prevented that slowing down the breathing guidance signal frequency could have a counter effective effect. For example, the breathing guidance signal frequency could be relatively low while the heart coherence and/or heart rate variability is relatively high. For example, the breathing guidance signal frequency is lowered whereas the heart beat signal frequency increases. This may have a negative effect on the heart rate variability and/or the user may be too tense, for example because his or her breathing rhythm is too low. Therefore, in an embodiment, an algorithm is present that is configured such that when an increase in the heart beat signal frequency and/or heart rate variability is measured after N is increased, N is decreased until the measured heart rate signal frequency and/or heart rate variability is stable, i.e. showing no significant characteristic changes during a significant period, or decreased. For example, when the user 2 experiences stress or experiences difficulty in maintaining a low breathing frequency, the average heart rate may go up and/or the heart rate variability may change undesirably. Since this will have a counter-effective effect the guidance tool 1 will allow the user 2 to decrease the breathing rhythm to a more convenient level, or use the N value that was used before it was increased, until a satisfactory heart rate and/or heart rate variability is measured. When a certain heart coherence and/or heart rate variability is again reached, after a certain period, N may be increased again, for example. In this way, a self learning breathing guide 1 is provided. In other words, a breathing guide 1 is provided that is adapted to the user and so that the user will improve his heart rate variability and/or heart coherence in a relatively intuitively fashion. Advantageously, N is continuously and automatically increased and/or decreased such that the user's effort to follow the guidance signals are relatively little. For example, said little effort can be indicated by a relatively stable or increasing heart rate variability.

According to the same principle, when the heart rate variability increases, which is generally positive, it may be more secure and/or have more enduring effects if the breathing signal frequency is stabilized or even slightly increased. This may help the user 2 better to keep control over him or herself and will not demand too much of the user 2 and/or will avoid negative emotions. In this way, also a steady course can be set for the user 2 and the heart coherence and/or variability may be improved steadily and in a relatively intuitive way.

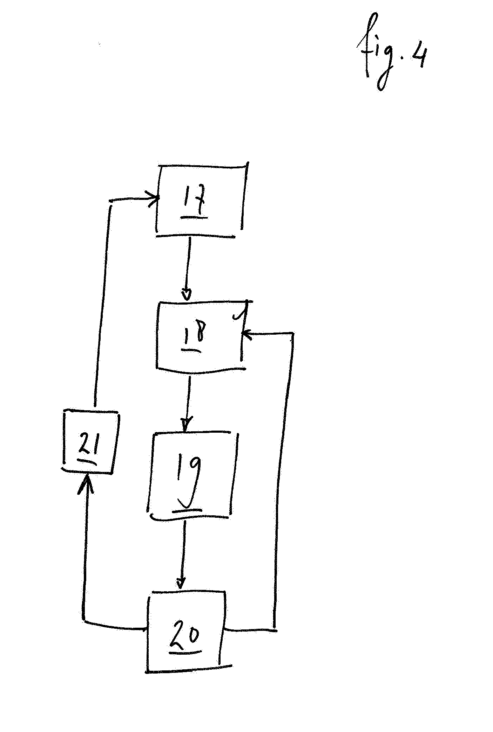

A possible flow chart that indicates a self-adjusting breathing guide is illustrated in Figure 4, wherein in step 17 a first heart beat signal frequency (HR1) is measured and in step 18, N is increased, for example to N + 1. In this step 18, the breathing guidance signal frequency is decreased as a consequence of the increased N. As a consequence the user 2 applies a slower breathing rhythm and the average heart rate (and hence the heart beat signal frequency) should may drop. In step 19, a second heart beat signal frequency is measured (HR2). In step 20 it is determined whether the first heart beat signal frequency is larger than the second heart beat signal frequency (HR1>HR2) or smaller (HR2>HR1). If it is larger, a satisfactorily result has been achieved and after a period of time, N+1 may be increased again, to N+2, and the loop may start again at step 18. If the first heart beat signal frequency is lower than the second (HR1<HR2) then the heart rate has gone up while the breathing rhythm was slowed down or supposed to slow down. Probably the heart coherence and/or heart rate variability have gone down and the user 2 may increase his breathing rhythm again to start over and slowly reach a more optimal breathing rhythm. Therefore N is again in step 21 decreased and again the heart rate is measured as indicated in step 17. In such a way, a self-adjusting system is reached.

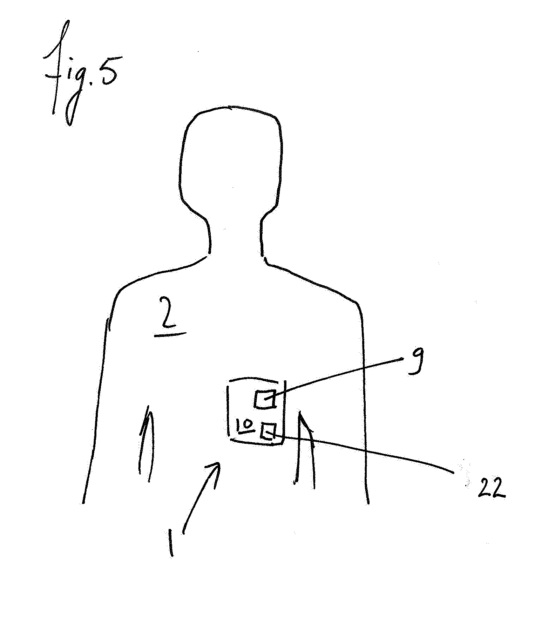

A user 2 may for example be guided by a breathing guide 1 during relatively long periods. Hence in an embodiment, the breathing guide 1 as a non-obtrusive, easy to wear breathing guide 1, see for example Figure 5. For example, the breathing guide can be worn under and/or as clothing by a person. For example, the breathing guide 1 can be integrated with clothes, a piece of cloth, a ribbon, a plaster, a piece of bandage, a patch 10, etc. The breathing guide 1 may have connecting means such that it is connectable to the body of a human or animal or may be worn by a human or animal. Advantageously the guide 1 can be configured to be of light weight such that it is hardly noticed and can be conveniently carried during long periods. An embodiment that may be worn in a convenient way, for example as shown in Figure 5, may be combined with above mentioned self adjusting features of N. In this way, the breathing guide 1 can be worn and the heart coherence and/or heart rate variability can be improved in an intuitive and automated way for the user 2. The user 2 may for example be occupied with other or related tasks, e.g. such as doing the daily job, reading, jogging, listening to music, yoga, practicing any sport, etc., while he or she is breathing synchronous with the breathing guidance signals that are given by the breathing guide 1.

Also, the user interface 6 may comprise a vibration actuator 9 that may directly or indirectly vibrate against the body of the user 2. The vibration actuator 9 is preferably configured such as to signal the user 2 when to breathe in and/or out in an unobtrusive way. For example, the vibration signals may be given while the user 2 is talking and the user's heart beat may be synchronous with his or her breathing while he or she is performing other tasks. Furthermore the breathing guide 1 may for example comprise a user setting interface 22 with which the settings of the breathing guide 1 can be adjusted. For example, N can be adjusted manually with the user setting interface 22. Obviously, means for connecting the breathing guide 1 to a computer and/or power supply 7 may be provided.

With the various embodiments as described above, a convenient breathing guide can be achieved wherein the user's breathing is guided in an intuitive way. The user 2 may then synchronize his or her breathing with the heart beats without being expressively conscious. With relatively little effort, a certain heart coherence and/or heart rate variability may be achieved, maintained and preferably improved.

In again other embodiments, the breathing guide 1 is integrated with a mobile device, such as for example a music player and/or a mobile communication device and/or a mobile computer. For example, computer program products and/or sensors 3 could be stored in and/or connected to these mobile devices, such that the functions of a breathing guide 1 as explained above can be achieved.

Also, in an embodiment the breathing guide 1 is coupled to and/or integrated with a second emotion measuring device, for example a device measuring tension, arousal and/or relaxation of a user 2 in an automated way. For this, the breathing guide 1 may comprise a second sensor and an algorithm configured to process a body parameter signal, such as for example skin resistance (e.g. by GSR measurement and/or EMG measurement) muscle tension and/or blood pressure measurement, for deriving information about the state of the user 2 in an other way than measuring the heart rate. Body parameter signals such as these are known to derive information concerning the state of the user 2. In this way through a combination of techniques, i.e. heart rate measurement as well as measuring another body parameter, feedback about the state of the user 2 can be obtained in an automated way. Through this combination, a more reliable reading of the state of the user 2 can be obtained and breathing guidance can be adjusted to that reading for guiding the user towards an improved heart coherence and/or heart rate variability. For example, emotion measuring techniques may include GSR measurements, EMG measurement, muscle tension measurement, blood pressure measurement, but are not limited to those.

It shall be obvious that the invention is not limited in any way to the embodiments that are represented in the description and the drawings. Many variations and combinations are possible within the framework of the invention as outlined by the claims. Combinations of one or more aspects of the embodiments or combinations of different embodiments are possible within the framework of the invention. All comparable variations are understood to fall within the framework of the invention as outlined by the claims.

## Claims

1. Computer program product for guiding the breathing of a user, which when executed on a computer controls the computer to process received heart beat signals and to provide for breathing guidance signals via a user interface, wherein the computer program product comprises an algorithm that is configured to synchronize the breathing guidance signals with received heart beat signals for the purpose of guiding the user towards an improved heart rate variability and/or heart coherence.

2. Computer program product according to claim 1, wherein the algorithm is configured to calculate a breathing guidance signal frequency by deriving a heart beat signal frequency and dividing the heart beat signal frequency by a number, defined by N.

3. Computer program product according to claim 1 or 2, wherein the heart beat signal frequency is the average heart beat signal frequency calculated over a number of previously received heart beat signals, for example between the last twenty and the last three heart beat signals.

4. Computer program product according to any one of the preceding claims, wherein the algorithm is configured to indicate the breathing guidance signal on every Nth heart beat signal, wherein N is an integer number.

5. Computer program product according to any one of the preceding claims, wherein the breathing guidance signal frequency is double the breathing rhythm and wherein N is such that when the computer program product is executed, the breathing rhythm is between 10 and 18 breaths per minute, preferably approximately 15 breaths per minute.

6. Computer program product according to any one of the preceding claims, configured to, when the computer program product is executed on a computer, store a heart beat signal pattern that corresponds to a heart rate, and calculate a breathing guidance signal frequency from the heart beat signal pattern, and to calculate N such that the breathing guidance signals are synchronized with the peaks and valleys of the heart beat signal pattern of the user.

7. Computer program product according to any one of the preceding claims, wherein the algorithm is configured such that when executed on a computer, N is gradually increased in time, such that the breathing guidance signal frequency is gradually decreased in time.

8. Computer program product according to any one of the preceding claims, wherein the algorithm is configured such that when executed on a computer the value of N is increased and stored in steps and a time interval between steps is stored, and wherein at a later time for the same time interval between steps, N is increased by a larger value as the stored value.

9. Computer program product according to any one of the preceding claims, wherein the algorithm is configured such that when executed on a computer the value of N is increased and stored in steps and a time interval between steps is stored, and wherein at a later time the time interval between the steps of increasing N is shortened.

10. Computer program product according to any one of the preceding claims, wherein the algorithm is configured such that changes in the heart beat signal frequency are measured and when an increase in the heart beat signal frequency is measured after N has been increased, N is decreased until the measured heart beat signal frequency is stable or decreased.

11. Computer program product according to any one of the preceding claims, wherein the algorithm is configured such that a change in the heart rate variability is measured and an when an increase in the heart rate variability is measured after N is increased, N is stabilized or decreased, for ensuring a relatively steady increase in the heart rate variability.

12. Computer program product according to any one of the preceding claims, wherein the algorithm is configured to process an other body parameter signal next to the heart beat signal, for deriving information about the state of the user, for example using GSR measurement, EMG measurement, muscle tension measurement and/or blood pressure measurement techniques.

13. Breathing guide for guiding the breathing of a user, provided with a heart beat sensor configured to convert a human or animal heart beat into heart beat signals, a processing circuit configured to process heart beat signals and breathing guidance signals, a user interface configured to indicate breathing guidance signals and a storage arrangement configured to store a computer program product according to any one of the preceding claims which controls the breathing guide.

14. Breathing guide according to claim 13, wherein the user interface comprises a vibration actuator arranged for vibrating against a human or animal body.

15. Breathing guide according to claim 13 or 14, wherein the breathing guide is arranged to be worn under and/or as clothing by a person and/or having connecting means to connect to the body.

16. Breathing guide according to any one of claims 13 - 15, that is coupled to and/or integrated with a second emotion measuring device that measures body parameter signals in an automated way.

17. Method for guiding the breathing of a human or animal, wherein heart beat signals are measured in an automated way, wherein breathing guidance signals that are synchronized with heart rate signals are given in an automated way for the purpose of guiding the user towards an improved heart coherence and/or heart rate variability.

18. Method according to claim 17, wherein a breathing guidance frequency is calculated by dividing the heart beat signal frequency, with a number, defined by N.

19. Method according to claim 17 or 18, wherein an average heart beat signal frequency is calculated over a previous set of received heart beat signals, for example between the previous twenty and the previous three heart beat signals.

20. Method according to any one of claims 17 - 19, wherein the breathing guidance signal is given approximately on every Nth heart beat signal.

21. Method according to any one of claims 17 - 20, wherein N is chosen such that the breathing guidance frequency is between 10 and 18 breaths per minute, preferably close to 15 breaths per minute.

22. Method according to any one of claims 17 - 21, wherein N is gradually increased in time, such that the breathing guidance frequency is gradually decreased.

23. Method according to any one of claims 17 - 22, wherein when an increase in the heart rate signal frequency is measured after N is increased, N is decreased until the heart rate signal frequency is determined to be stable or decreased.

24. Method according to any one of claims 17 - 23, wherein when an increase in the heart rate variability is measured after N has been increased, N is stabilized or decreased, for ensuring a relatively steady increase in the heart rate variability.

25. Method according to any one of claims 17 - 24, wherein the value of N is increased and stored in steps and the time interval between steps is stored, and wherein at a later time for the same time interval between steps, N is increased by a larger value as the stored value.

26. Method according to any one of claims 17 - 25, wherein the value of N is increased and stored in steps and a time interval between steps is stored, and wherein at a later time the time interval between the steps of increasing N is shortened.

27. Method according to any one of claims 17 - 25, wherein next to the heart beat signals other body parameter signals are measured and evaluated to guide the user towards an improved heart coherence and/or heart rate variability.

28. Breathing guide for guiding the breathing of a user, provided with a heart beat sensor configured to convert a human or animal heart beat into heart beat signals, a storage arrangement configured to store an algorithm, a processing circuit configured to process heart beat signals and breathing guidance signals, and a user interface configured to indicate a breathing guidance signal, wherein the algorithm is configured to convert the heart beat signals into breathing guidance signals and to synchronize the breathing guidance signals with the processed heart rate signals for the purpose of guiding the user towards an improved heart coherence and/or heart rate variability.
